# FASCICULE DE BREVET EUROPEEN

(11) **EP 0 922 018 B1**
(45) Date de publication et mention de la délivrance du brevet: **20.09.2000**
(21) Numéro de dépôt: 96944087.4
(22) Date de dépôt: 27.12.1996
(51) Int. Cl.: C07C 11/09

(54) **PROCEDE DE PRODUCTION D'ISOBUTENE DE HAUTE PURETE A PARTIR D'UNE COUPE C4 CONTENANT DE L'ISOBUTENE ET DU BUTENE-1**
VERFAHREN ZUR HERSTELLUNG VON HOCHREINEM ISOBUTEN AUS EINEM C4-SCHNITT DER ISOBUTEN UND BUTEN-1 ENTHÄLT
METHOD FOR PRODUCING HIGH PURITY ISOBUTYLENE FROM A BUTANE PLUS FRACTION CONTAINING ISOBUTYLENE AND BUTYLENE-1

(30) Priorité: 08.08.1996 FR 9610101
(43) Date de publication de la demande: 16.06.1999
(73) Titulaire: INSTITUT FRANCAIS DU PETROLE, 92500 Rueil Malmaison (FR)
(72) Inventeur: DORBON, Michel, F-69002 Lyon (FR); DIDILLON, Blaise, F-92500 Rueil Malmaison (FR); VILTARD, Jean-Charles, F-26000 Valence (FR); COSYNS, Jean, F-78580 Maule (FR); CAMERON, Charles, F-75005 Paris (FR); UNTERBERG, Heinz, D-41540 Dormagen (DE); SCHÜMMER, Günter, D-50259 Pullheim (DE); BROWN, William, Martin, Sarnia, Ontario N7S 3V5 (CA)
(86) Numéro de dépôt international: FR9602086
(87) Numéro de publication internationale: WO9806684

(56) Documents cités:
- EP-A- 0 170 182
- EP-A- 0 380 374
- FR-A- 2 528 033
- US-A- 5 087 780

## Description

L'invention concerne un procédé de traitement d'une coupe d'hydrocarbures comportant essentiellement des hydrocarbures oléfiniques avant 4 atomes de carbone par molécule et comportant de l'isobutène, ainsi que du butène-1 et des butènes-2 en rapport correspondant sensiblement à l'équilibre thermodynamique, ledit procédé comprenant le passage de ladite coupe dans une zone de distillation associée à une seconde zone réactionnelle d'hydroisomérisation, ledit procédé étant caractérisé en ce que la zone d'hydroisomérisation est au moins en partie externe à la zone de distillation. Un tel procédé permet la production d'isobutène de haute pureté.

L'isobutène destiné à la polymérisation doit avoir un niveau de pureté supérieur à 99% et ne plus contenir que des traces de butène-1 et de butènes-2 (quelques centaines de parties par million en poids, ppm). En effet, si le taux d'impureté dans l'isobutène est trop élevé, les polymères obtenus sont de moins bonne qualité et le rendement de la polymérisation est plus faible. Il est donc nécessaire d'éliminer d'une coupe d'hydrocarbures contenant de l'isobutène les autres hydrocarbures oléfiniques, comportant 4 atomes de carbone par molécule. Le butène-1 et l'isobutène ayant des points d'ébullition très voisins, il n'est pas possible de les séparer par distillation, à moins de mettre en oeuvre des moyens considérables. Les autres hydrocarbures oléfiniques ayant 4 atomes de carbone par molécule peuvent être séparés de l'isobutène par distillation.

Le principal problème qui se pose pour produire de l'isobutène de haute pureté est donc la séparation du butène-1 de l'isobutène. Pour effectuer cette séparation, plusieurs voies sont envisageables.

La première voie consiste en une extraction à l'acide sulfurique : l'isobutène est hydraté sélectivement et régénéré ensuite par traitement de la phase aqueuse. Si la température et la concentration sont bien contrôlées, ce procédé permet d'obtenir de l'isobutène de bonne pureté. Cependant, le rendement n'excède habituellement pas 90%, l'extraction n'étant pas complète, et il se forme des dimères et oligomères, conduisant à la formation de boues acides toxiques.

La deuxième voie consiste en un craquage de l'éther méthylique de l'alcool tertiobutylique (MTBE) : l'isobutène est extrait de la coupe C₄ par réaction avec du méthanol afin de former du MTBE. Le MTBE est alors craqué en méthanol et isobutène sur un catalyseur acide. Le rendement de récupération est généralement d'environ 96%. L'isobutène produit est de bonne pureté, mais il doit être débarrassé du diméthyléther qui peut se former durant le craquage.

La troisième voie envisageable est la déshydratation de l'alcool butylique tertiaire (ABT). Dans l'opération précédente, le méthanol peut être remplacé par l'eau, ce qui conduit à la production d'ABT. L'isobutène est ensuite récupéré par déshydratation de l'ABT. Cette voie n'est pratiquement pas utilisée, essentiellement parce que l'ABT est très lié au marché de l'oxyde de propylène peut, selon les procédés, être un sous-produit de l'oxyde de propylène.

Le brevet US-A-5.177.283 décrit un procédé de conversion d'hydrocarbures, qui comprend le passage de la charge dans une zone de fractionnement, l'effluent de tête étant riche en un des réactifs et l'effluent de fond étant riche en produit de la réaction, ledit procédé étant tel que l'on procède au soutirage latéral d'un flux liquide, qui est passé avec un flux gazeux riche en hydrogène dans une zone de réaction catalytique, ce qui permet d'obtenir un effluent de zone de réaction comprenant un des réactifs et le produit de la réaction, une fraction de la partie gazeuse dudit effluent étant recyclée en zone de réaction, la partie liquide dudit effluent étant renvoyée en zone de fractionnement, généralement à proximité du soutirage. Il n'est pas fait mention de la réaction d'hydroisomérisation du butène-1 en butènes-2.

Le brevet britannique GB 1 595 526 décrit un procédé de production d'isooléfines, notamment d'isobutène, dans lequel une charge contenant des butènes normaux et de l'isobutène est séparée en deux fractions. La fraction riche en isobutène et comprenant du butène-1 est envoyée dans un réacteur d'isomérisation permettant d'isomériser le butène-1 en butène-2, puis l'effluent du réacteur est en partie renvoyé dans la colonne de fractionnement, et en partie récupéré en tant que produit riche en isobutène. La charge du réacteur d'isomérisation est au moins en partie voire totalement gazeuse, ainsi qu'il est indiqué dans les exemples. Par ailleurs, la partie de l'effluent issue du réacteur d'isomérisation qui est renvoyée dans la colonne de fractionnement n'est pas réintroduite dans la zone de fractionnement elle-même, mais dans la zone de reflux.

Le procédé selon l'invention permet de produire de l'isobutène de haute pureté au moindre coût et avec un excellent rendement, à partir d'une coupe C₄ oléfinique contenant au moins de l'isobutène, ainsi que du butène-1 et des butènes-2 en rapport correspondant sensiblement à l'équilibre thermodynamique, généralement issue d'un procédé de vapocraquage, telle que la coupe C₄ brute ou le raffinat-1 (obtenu après extraction du butadiène de la coupe C₄ brute), ou de craquage catalytique. Le procédé selon l'invention est caractérisé par l'intégration d'opérations de distillation et d'hydroisomérisation agencées et opérées de manière à minimiser le coût d'investissement du procédé, à maximiser la conversion du butène-1 en butènes-2 et à minimiser l'hydrogénation de l'isobutène en isobutane, afin de maximiser le rendement en isobutène. Ainsi, le procédé selon l'invention peut réaliser au moins partiellement l'hydrogénation sélective des composés polyinsaturés le plus souvent dièniques ou acétyléniques tels que le butadiène, le vinylacétylène, le méthylacétylène et l'éthylacétylène, quand ces composés sont présents dans la charge, et l'hydroisomérisation du butène-1 en butènes-2 (cis et trans). Les butènes-2 produits de cette hydrogénation et de cette hydroisomérisation peuvent alors être séparés de l'isobutène par distillation, ce qui n'est pas le cas du butène-1. Par rapport aux autres procédés cités précédemment, le procédé selon l'invention a l'avantage d'avoir un rendement en isobutène élevé, toujours supérieur à 90%, généralement supérieur à 95 %, et de façon préférée supérieur à 98%, et de ne pas produire de sous-produits oxygénés.

Le procédé selon l'invention est un procédé de traitement d'une charge, comprenant en majeure partie des hydrocarbures oléfiniques comportant 4 atomes de carbone par molécule, dont de l'isobutène, ainsi que du butène-1 et des butènes-2 en rapport correspondant sensiblement à l'équilibre thermodynamique, dans lequel on traite ladite charge dans une zone de distillation, comportant généralement une zone d'épuisement et une zone de rectification, associée à une zone réactionnelle d'hydroisomérisation, ledit procédé étant caractérisé en ce que la zone d'hydroisomérisation Ri2 est au moins en partie externe à la zone de distillation.

La charge de la partie externe de la zone réactionnelle est généralement prélevée à la hauteur d'un niveau de prélèvement de la zone de distillation et représente au moins une partie, de préférence la majeure partie du liquide (reflux) coulant dans la zone de distillation, de préférence coulant dans la zone de rectification, et de façon encore plus préférée coulant à un niveau intermédiaire de la zone de rectification, l'effluent de la zone réactionnelle étant au moins en partie, de préférence en majeure partie, réintroduit dans la zone de distillation à au moins un niveau de réintroduction, situé généralement à proximité du niveau de prélèvement, c'est-à-dire sensiblement à la hauteur ou sensiblement au-dessus ou sensiblement au-dessous, le plus souvent sensiblement à la hauteur ou sensiblement au-dessus, d'un niveau de prélèvement, de préférence dudit niveau de prélèvement, c'est-à-dire situé généralement à une distance dudit niveau correspondant à une hauteur comprise entre 0 et 4 plateaux théoriques au-dessus ou au-dessous d'un niveau de prélèvement, de préférence situé sensiblement à la hauteur ou sensiblement au-dessus dudit niveau de prélèvement, de manière à assurer la continuité de la distillation. Le procédé selon l'invention permet de sortir en tête de la zone de distillation un effluent riche en isobutène, généralement de haute pureté, et en fond de zone de distillation un effluent appauvri en isobutène.

Le procédé selon l'invention permet la production d'isobutène de haute pureté. Toute zone réactionnelle d'hydroisomérisation est généralement telle que toute réaction d'hydroisomérisation est réalisée en présence d'un catalyseur d'hydroisomérisation et d'un flux gazeux comprenant, de préférence en majeure partie, de l'hydrogène.

La charge qui alimente la zone de distillation est introduite dans ladite zone généralement au moins à un niveau de ladite zone, de préférence principalement à un seul niveau de ladite zone. Elle est en rapport correspondant sensiblement à l'équilibre thermodynamique butène-1 - butène-2 à l'introduction. Une des mises en oeuvre préférée du procédé selon l'invention comprend l'obtention de ladite charge à partir d'une coupe comprenant en majeure partie des hydrocarbures oléfiniques comportant 4 atomes de carbone par molécule par traitement de ladite coupe dans une première zone d'hydroisomérisation, généralement indépendante de la partie externe de la zone réactionnelle d'hydroisomérisation associée à la zone de distillation, la majeure partie de l'effluent de ladite première zone d'hydroisomérisation servant alors de charge, principale ou secondaire selon les définitions données ci-après dans le texte, qui alimente la zone de distillation. Si ladite charge comprend des composés polyinsaturés, le plus souvent diéniques et/ou acétyléniques, lesdits composés sont de préférence transformés en butènes par la première zone d'hydroisomérisation avant l'introduction dans la zone de distillation. Niais tout autre technique permettant d'obtenir, à partir d'une coupe comprenant en majeure partie des hydrocarbures oléfiniques en C₄ dont du butène-1 et de l'isobutène, une charge où le butène-1 et les butènes-2 sont en rapport correspondant sensiblement à l'équilibre thermodynamique est aussi envisageable dans le cadre de l'invention.

La zone de distillation comprend généralement au moins une colonne munie d'au moins un interne de distillation choisi dans le groupe formé par les plateaux simples, les plateaux à multi-déversoirs, les garnissages en vrac et les garnissages structurés, ainsi qu'il est connu de l'homme du métier, de telle sorte que l'efficacité globale totale est au moins égale à cinq étages théoriques. Dans les cas connus de l'homme du métier où la mise en oeuvre d'une seule colonne pose des problèmes, on préfère généralement scinder ladite zone de façon à utiliser finalement au moins deux colonnes qui, mises bout à bout, réalisent ladite zone, c'est-à-dire que la zone de rectification, la zone réactionnelle éventuelle et la zone d'épuisement se répartissent sur les colonnes.

La première zone réactionnelle d'hydroisomérisation éventuelle, située en amont de la zone de distillation, réalise au moins partiellement l'hydrogénation sélective des composés polyinsaturés, le plus souvent diéniques tel que le butadiène, en plus de l'hydroisomérisation d'au moins une partie du butène-1 en butènes-2. Elle comprend généralement au moins un lit catalytique d'hydroisomérisation comprenant un catalyseur d'hydroisomérisation, de préférence de 1 à 4 lit(s) catalytique(s) ; dans le cas où au moins deux lits catalytiques se trouvent incorporés dans ladite zone réactionnelle, ces deux lits sont de préférence répartis dans au moins deux réacteurs, répartis en série ou en parallèle, de préférence en série. Par exemple ladite première zone réactionnelle comprend un seul réacteur dans lequel se trouve au moins un, et de préférence un seul, lit catalytique. Une des mises en oeuvre préférées du procédé de la présente invention est telle que ladite première zone réactionnelle comprend deux réacteurs généralement répartis en série comportant chacun au moins un, de préférence un seul, lit catalytique. Lorsque ladite zone réactionnelle comprend au moins deux réacteurs, le recyclage éventuel d'au moins une partie de l'effluent d'au moins un des réacteurs compris dans ladite première zone réactionnelle dans ladite zone, est effectué généralement à l'entrée d'un réacteur, de préférence dudit réacteur, de préférence avant l'injection d'un composé gazeux comprenant de l'hydrogène. Il est aussi possible de procéder à un recyclage autour de ladite première zone elle-même, c'est-à-dire généralement à l'entrée du premier réacteur de ladite zone, de préférence avant l'injection du composé gazeux comprenant de l'hydrogène ; par exemple au moins une partie de l'effluent du second réacteur est recyclée vers l'entrée du premier réacteur. Ceci permet avantageusement d'abaisser la teneur en composés polyinsaturés dans l'effluent de ladite première zone réactionnelle.

Les conditions opératoires de la première zone d'hydroisomérisation, lorsqu'elle est présente, sont généralement les suivantes : le catalyseur est identique au catalyseur de la zone d'hydroisomérisation qui sera décrit ci-après. La pression est généralement comprise entre 4 et 40 bar (1 bar = 0,1 MPa), de préférence entre 6 et 30 bar. La température est généralement comprise entre 10 et 150°C, de préférence entre 20 et 100°C. Le rapport molaire H₂/hydrocarbures est généralement ajusté de façon à obtenir une conversion pratiquement totale des composés polyinsaturés tel que le butadiène et une isomérisation suffisante du butène-1 en butènes-2 avec formation limitée d'alcanes.

La zone réactionnelle d'hydroisomérisation associée à la zone de distillation comprend généralement au moins un lit catalytique d'hydroisomérisation comprenant un catalyseur d'hydroisomérisation, de préférence de 2 à 6, de façon encore plus préférée de 2 à 4 lits catalytiques ; dans le cas où au moins deux lits catalytiques se trouvent incorporés dans la zone de distillation, ces deux lits sont de préférence séparés par au moins un interne de distillation.

Ladite zone réactionnelle d'hydroisomérisation réalise au moins partiellement l'hydroisomérisation d'au moins une partie, de préférence la majeure partie, du butène-1 présent dans sa charge en butènes-2 (cis et trans), généralement de telle façon que la teneur en butène-1 de l'effluent de tête de la zone de distillation soit au maximum égale à une certaine teneur. De préférence, le procédé selon l'invention est tel que le rapport molaire butène-1 sur isobutène dans l'effluent de tête de la zone de distillation est inférieur à 2 x 10⁻³, de préférence inférieur à 1 x 10⁻³, et de façon encore plus préférée inférieur à 5 x 10⁻⁴.

Une des mises en oeuvre préférées du procédé selon l'invention comprend l'alimentation de la zone de distillation, en plus de l'alimentation en la charge principale, en une charge dite secondaire (par rapport à la charge principale), qui provient ou non d'une zone réactionnelle d'hydroisomérisation telle que la première zone réactionnelle éventuelle d'hydroisomérisation, indépendamment ou non de l'alimentation de la zone de distillation en la charge principale. La charge secondaire est généralement une coupe oléfinique C₄ contenant au moins de l'isobutène, ainsi que du butène-1 et des butènes-2 en rapport correspondant sensiblement à l'équilibre thermodynamique, et est généralement issue d'un procédé de vapocraquage, telle que la coupe C₄ brute ou le raffinat-1, ou de craquage catalytique ; généralement et de façon préférée, la charge secondaire est une coupe oléfinique C₄ essentiellement exempte de composés polyinsaturés et sa teneur en butène-1 est inférieure à la teneur en butène-1 de la charge principale. Si la teneur en composés insaturés de la charge secondaire est élevée, ladite charge est de préférence traitée dans une zone d'hydrogénation sélective avant son entrée dans la zone de distillation.

Lorsque la charge principale est introduite en un seul niveau d'introduction, la charge secondaire est généralement introduite dans la zone de distillation en au moins un niveau d'introduction, de préférence en un seul niveau d'introduction, ledit niveau d'introduction dépendant de la composition de ladite charge secondaire. Ainsi, dans un premier exemple la charge secondaire peut être très riche en isobutène et contenir moins de 1,5 fois de butène-1 que la charge principale n'en contient, auquel cas la charge secondaire est de préférence introduite en un seul niveau situé généralement au-dessus du niveau d'introduction de la charge principale. Ou bien dans un second exemple la charge secondaire peut être pratiquement exempte de butène-1, auquel cas la charge secondaire est de préférence introduite en un seul niveau situé généralement au-dessous du niveau d'introduction de la charge principale. Il est aussi possible de procéder au mélange éventuel de la charge principale, avant son entrée en zone de distillation, et de la charge secondaire.

Le procédé selon l'invention est tel que la zone réactionnelle d'hydroisomérisation associée à la zone de distillation est au moins en partie externe à la zone de distillation. Généralement, le procédé selon l'invention comprend de 1 à 6, de préférence de 2 à 4 niveau(x) de prélèvement qui alimente(nt) la partie externe de ladite zone d'hydroisomérisation. Une partie de la partie externe de ladite zone d'hydroisomérisation qui est alimentée par un niveau de prélèvement donné, si ladite partie externe comprend au moins deux niveaux de prélèvement, comprend généralement au moins un réacteur, de préférence un seul réacteur. Si ladite partie de la partie externe comprend au moins deux lits catalytiques répartis dans au moins deux réacteurs, lesdits réacteurs sont disposés en série ou en parallèle et chacun desdits réacteurs est alimenté par un seul niveau de prélèvement, de préférence associé à un seul niveau de réintroduction, ledit niveau de prélèvement étant distinct du niveau de prélèvement qui alimente l'(es) autre(s) réacteur(s).

Le procédé selon l'invention est généralement tel que la charge de toute partie de la zone réactionnelle d'hydroisomérisation associée à la zone de distillation, qu'elle soit interne ou éventuellement externe, est prélevée à la hauteur d'un niveau de prélèvement et représente au moins une partie, de préférence la majeure partie, du liquide (reflux) coulant dans la zone de distillation, de préférence coulant dans la zone de rectification et de façon encore plus préférée coulant à un niveau intermédiaire de la zone de rectification, l'effluent de la zone réactionnelle d'hydroisomérisation étant au moins en partie, de préférence en majeure partie, réintroduit dans la zone de distillation, de manière à assurer la continuité de la distillation.

Le procédé selon l'invention permet d'isomériser une grande partie du butène-1 en butènes-2 à l'extérieur de la zone de distillation, éventuellement dans des conditions de pression et/ou de température différentes de celles utilisées dans la colonne. De préférence, la température à l'entrée (respectivement à la sortie) du niveau de prélèvement qui alimente un lit catalytique de la partie de la zone d'hydroisomérisation associée à la zone de distillation située à l'extérieur de la zone de distillation, est sensiblement semblable, c'est-à-dire que l'écart est sensiblement inférieur à 10°C par rapport à la température à la hauteur du niveau de prélèvement (respectivement du niveau de réintroduction). De même, on peut avantageusement effectuer la réaction d'hydroisomérisation dans ladite partie de la zone réactionnelle située à l'extérieur de la zone de distillation à une pression plus élevée que celle utilisée à l'intérieur de la zone de distillation. Cette augmentation de pression permet aussi une dissolution accrue du flux gazeux contenant de l'hydrogène dans la phase liquide contenant le butène-1 à hydroisomériser.

Le procédé selon l'invention est généralement tel que l'écoulement du liquide à hydroisomériser est co-courant à l'écoulement du flux gazeux comprenant de l'hydrogène, pour tout lit catalytique de la partie externe de la zone d'hydroisomérisation associée à la zone de distillation et pour tout lit catalytique de la première zone d'hydroisomérisation éventuelle.

Une des mises en oeuvre préférées du procédé selon l'invention comprend l'utilisation de la technique dite de "pump-around", c'est-à-dire de pompage en boucle, qui consiste à faire passer à l'extérieur de la zone de distillation une partie, de préférence la majeure partie, du liquide (reflux) par un facteur compris entre 0,5 et 1,5, de préférence compris entre 0,75 et 1,3, c'est-à-dire que le débit d'un lit catalytique de la partie externe de la zone d'hydroisomérisation associée à la zone de distillation, ledit lit étant alimenté à un niveau de prélèvement par au moins une partie de l'effluent liquide (reflux) coulant sur le plateau de distillation associé audit niveau de prélèvement (c'est-à-dire sur lequel on prélève ladite partie d'effluent liquide) et par au moins une partie du liquide correspondant au recyclage de l'effluent dudit lit sensiblement au-dessus ou sensiblement au-dessous ou sensiblement à la même hauteur que ledit niveau de prélèvement, est inférieur ou supérieur à 1 fois le débit de liquide coulant sur ledit plateau, de manière encore plus préférée égal à une fois, c'est-à-dire généralement du même ordre de grandeur que le débit de liquide coulant sur ledit plateau. Ainsi, le débit de charge d'un réacteur de la partie externe de la zone d'hydroisomérisation associée à la zone de distillation au niveau de prélèvement alimentant ledit réacteur, est de préférence supérieur à 1 fois le débit de liquide coulant sur le plateau associé audit niveau de prélèvement.

Selon un des modes de réalisation du procédé selon l'invention, la zone d'hydroisomérisation associée à la zone de distillation est à la fois partiellement incorporée dans la zone de distillation, c'est-à-dire interne à la zone de distillation, et partiellement externe à la zone de distillation. Selon un tel mode de réalisation, la zone d'hydroisomérisation comprend au moins deux, de préférence au moins trois lits catalytiques, au moins un lit catalytique étant interne à la zone de distillation, et au moins un lit catalytique étant externe à la zone de distillation. Généralement, les hydrocarbures liquides dont le butène-1 à isomériser, soit partiellement, soit totalement, circulent d'abord dans la partie externe de ladite zone d'hydroisomérisation puis dans la partie interne de ladite zone d'hydroisomérisation. Pour la partie de la zone réactionnelle interne à la zone de distillation, le prélèvement de liquide (reflux) est fait naturellement par écoulement dans la partie de la zone réactionnelle interne à la zone de distillation, et la réintroduction de liquide en zone de distillation se fait aussi naturellement par écoulement du liquide à partir de la partie de la zone réactionnelle interne à la zone de distillation. De plus, le procédé selon l'invention est de préférence tel que l'écoulement du liquide contenant le réactif, le butène-1, est co-courant ou contre-courant à l'écoulement du flux gazeux comprenant de l'hydrogène, pour tout lit catalytique de la partie interne éventuelle de la zone d'hydroisomérisation associée à la zone de distillation.

Selon un autre des modes de réalisation préférés du procédé selon l'invention, indépendant du mode de réalisation précédent, la zone d'hydroisomérisation associée à la zone de distillation est en totalité externe à la zone de distillation. Elle a alors les caractéristiques de la partie externe de la zone d'hydroisomérisation associée à la zone de distillation du mode de réalisation précédent.

Pour la réalisation de l'hydroisomérisation selon le procédé de l'invention, le rapport molaire H₂/hydrocarbures entrant dans la zone réactionnelle associée à la zone de distillation est au moins égal à 10⁻⁵. Ce rapport molaire peut-être optimisé de telle manière que d'une part tout l'hydrogène soit consommé dans les réactions d'hydroisomérisation afin d'éviter un dispositif de récupération d'hydrogène en sortie de zone réactionnelle, d'autre part de minimiser les réactions parasites d'hydrogénation de l'isobutène, afin de maximiser le rendement du procédé en isobutène et enfin de telle manière qu'il y ait suffisamment d'hydrogène tout le long de la zone réactionnelle pour que la réaction d'hydroisomérisation du butène-1 en butènes-2 puisse se faire. Cependant, si les conditions sont telles qu'il y ait excès d'hydrogène, l'hydrogène en excès peut être avantageusement récupéré. Par exemple, l'hydrogène en excès qui sort en tête de la zone de distillation peut être récupéré, puis comprimé et réutilisé dans la zone réactionnelle d'hydroisomérisation associée à la zone de distillation.

L'hydrogène, compris dans le flux gazeux utilisé dans le procédé de l'invention pour l'hydroisomérisation du butène-1 en butènes-2, que ce soit dans la première zone d'hydroisomérisation éventuelle ou bien dans la zone d'hydroisomérisation associée à la zone de distillation, provient généralement en majeure partie, de préférence en quasi totalité, de l'extérieur de la zone de distillation. Il peut provenir de toute source produisant de l'hydrogène à au moins 50% volume de pureté, de préférence au moins 80% volume de pureté, et de façon encore plus préférée au moins 90% volume de pureté. Par exemple, on peut citer l'hydrogène provenant des procédés de vapocraquage, de réformage catalytique, de P.S.A. (adsorption par alternance de pression) ou de génération électrochimique.

Lorsque la zone d'hydroisomérisation associée à la zone de distillation est au moins en partie incorporée à la zone de distillation, le catalyseur d'hydroisomérisation peut être disposé dans ladite partie incorporée suivant les différentes technologies proposées pour conduire des distillations catalytiques. Elles sont essentiellement de deux types.

Suivant le premier type de technologies, la réaction et la distillation procèdent simultanément dans le même espace physique, comme l'enseignent par exemple la demande de brevet WO-A-90/02.603, les brevets US-A-4.471.154, USA-4.475.005, US-A-4.215.011, US-A-4.307.254, US-A-4.336.407, US-A-4.439.350, US-A-5.189.001, US-A-5.266.546, US-A-5.073.236, US-A-5.215.011, US-A-5.275.790, US-A-5.338.517, US-A-5.308.592, US-A-5.236.663, US-A-5.338.518, ainsi que les brevets EP-B1-0.008.860, EP-B1-0.448.884, EP-B1-0.396.650 et EP-B1-0.494.550 et la demande de brevet EP-A1-0.559.511. Le catalyseur est alors généralement en contact avec une phase liquide descendante, générée par le reflux introduit au sommet de la zone de distillation, et avec une phase vapeur ascendante, générée par la vapeur de rebouillage introduite en fond de zone. Selon ce type de technologies, le flux gazeux comprenant de l'hydrogène nécessaire à la zone réactionnelle, pour la réalisation du procédé selon l'invention, pourrait être joint à la phase vapeur, de préférence sensiblement à l'entrée d'au moins un lit catalytique de la zone réactionnelle.

Suivant le second type de technologies, le catalyseur est disposé de telle façon que la réaction et la distillation procèdent généralement de manière indépendante et consécutive, comme l'enseignent par exemple les brevets US-A-4.847.430, US-A-5.130.102 et US-A-5.368.691, la vapeur de la distillation ne traversant pratiquement pas tout lit catalytique de la zone réactionnelle. Ainsi, si l'on utilise ce type de technologie, le procédé selon l'invention est généralement tel que l'écoulement du liquide à hydroisomériser est co-courant à l'écoulement du flux gazeux comprenant de l'hydrogène et tel que la vapeur de distillation n'est pratiquement pas en contact avec le catalyseur (ce qui se traduit généralement en pratique par le fait que ladite vapeur est séparée dudit liquide à hydroisomériser), pour tout lit catalytique de la partie interne de la zone d'hydroisomérisation. De tels systèmes comportent généralement au moins un dispositif de distribution de liquide qui peut être par exemple un répartiteur de liquide, dans tout lit catalytique de la zone réactionnelle. Néanmoins, dans la mesure où ces technologies ont été conçues pour des réactions catalytiques intervenant entre des réactifs liquides, elles ne peuvent convenir sans modification pour une réaction catalytique d'hydroisomérisation, pour laquelle l'un des réactifs, l'hydrogène, est à l'état gazeux.

Pour tout lit catalytique de la partie interne de la zone d'hydroisomérisation, il est donc généralement nécessaire d'adjoindre un dispositif d'introduction du flux gazeux comprenant de l'hydrogène, par exemple selon les techniques décrites ci-après. Ainsi, pour tout lit catalytique de la partie interne de la zone d'hydroisomérisation, la partie interne de la zone d'hydroisomérisation comporte au moins un dispositif de distribution de liquide, généralement situé au-dessous dudit lit catalytique, et au moins un dispositif d'introduction du flux gazeux, généralement situé au-dessous ou au sein dudit lit catalytique, de préférence dans ce dernier cas non loin du dispositif d'introduction du liquide. Selon une technique, le dispositif d'introduction du flux gazeux dans tout lit catalytique est identique au dispositif de distribution de liquide dans le lit catalytique, c'est-à-dire qu'il existe un moyen d'introduction du gaz dans le liquide en amont du dispositif de distribution de liquide (par rapport au sens de circulation du liquide). En pratique et en langage courant, ceci revient à réaliser un piquage du gaz dans le liquide en amont du moyen de distribution de liquide. Selon une autre technique, le dispositif d'introduction du flux gazeux est disposé sensiblement au niveau du dispositif de distribution de liquide, le gaz et le liquide étant introduits de façon séparée dans le lit catalytique. Selon cette autre technique, le dispositif d'introduction du flux gazeux est disposé au dessous ou au sein du lit catalytique de préférence non loin du dispositif de distribution de liquide.

Ainsi, lorsque la zone d'hydroisomérisation associée à la zone de distillation est au moins en partie interne à la zone de distillation, une des réalisations préférées du procédé selon l'invention est telle que le catalyseur de la partie interne de ladite zone d'hydroisomérisation est disposé dans ladite partie suivant le dispositif de base décrit dans le brevet US-A-5.368.691, aménagé de manière que tout lit catalytique de la partie interne de la zone d'hydroisomérisation soit alimenté par un flux gazeux comprenant de l'hydrogène, régulièrement distribué à sa base, par exemple selon l'une des trois techniques décrites ci-dessus. Suivant cette technologie, dans laquelle la zone de distillation comprend une seule colonne et dans laquelle la zone d'hydroisomérisation est en totalité interne à ladite colonne (ce qui diffère du procédé selon l'invention), le catalyseur compris dans tout lit catalytique, interne à la zone de distillation, est alors en contact avec une phase liquide ascendante, générée par le reflux introduit au sommet de la colonne de distillation, et avec le flux gazeux comprenant de l'hydrogène qui circule dans le même sens que le liquide; le contact avec la phase vapeur de la distillation étant évité en faisant transiter cette dernière par au moins une cheminée spécialement aménagée.

Lorsque la zone d'hydroisomérisation associée à la zone de distillation est au moins en partie interne à la zone de distillation, les conditions opératoires de ladite partie interne sont liées aux conditions opératoires de la distillation. La distillation est généralement conduite de manière à minimiser la quantité d'isobutène dans le produit de fond afin de maximiser le rendement du procédé en isobutène et de manière à minimiser la quantité de butènes-2 et butène-1 dans le produit de tête, afin d'avoir en tête de l'isobutène de haute pureté. Elle est réalisée sous une pression généralement comprise entre 2 et 30 bar, de préférence entre 4 et 15 bar, de façon encore plus préférée entre 4 et 10 bar (1 bar = 10⁵ Pa), avec un taux de reflux compris entre 1 et 30, et de préférence compris entre 5 et 20. La température de tête de zone de distillation est comprise généralement entre 0 et 200 °C et la température de fond de zone de distillation est comprise généralement entre 5 et 250 °C. Les températures de la zone de distillation peuvent être calculées à partir des pressions, ainsi qu'il est connu de l'homme du métier. La réaction d'hydroisomérisation est conduite dans des conditions qui sont le plus généralement intermédiaires entre celles établies en tête et en fond de zone de distillation, à une température comprise entre 20 et 150 °C, et de préférence comprise entre 40 et 80 °C, et à une pression comprise entre 2 et 30 bar, de préférence entre 4 et 15 bar, de façon encore plus préférée entre 4 et 10 bar. Le liquide soumis à l'hydroisomérisation est alimenté par un flux gazeux comprenant, de préférence en majeure partie, de l'hydrogène.

Dans la partie externe de la zone d'hydroisomérisation associée à la zone de distillation, le catalyseur est disposé dans tout lit catalytique suivant toute technologie connue de l'homme de métier dans des conditions opératoires (température, pression etc.) indépendantes ou non, de préférence indépendantes, des conditions opératoires de la zone de distillation.

Dans la partie de la zone d'hydroisomérisation associée à la zone de distillation externe à la zone de distillation, les conditions opératoires sont généralement indépendantes des conditions opératoires de la zone de distillation. Elles sont généralement les suivantes. La pression requise pour cette étape d'hydroisomérisation est généralement d'environ 1 à 40 bar absolus, de préférence d'environ 2 à 30 bar, et de façon encore plus préférée d'environ 4 à 25 bar. La température opératoire de ladite partie externe de ladite zone d'hydroisomérisation est généralement d'environ 20 à 150 °C, de préférence d'environ 40 à 100° C, et de façon préférée d'environ 40 à 80° C. La vitesse spatiale au sein de ladite zone d'hydroisomérisation, calculée par rapport au catalyseur, est généralement d'environ 1 à 100 et plus particulièrement d'environ 4 à 50 h⁻¹ (volume de charge par volume de catalyseur et par heure). Le débit d'hydrogène correspondant est tel que le rapport molaire H₂/hydrocarbures entrant dans la zone d'hydroisomérisation associée à la zone de distillation est de préférence au moins égal à 10⁻⁵. Ce rapport est le plus souvent d'environ 10⁻⁵ à environ 3 et très fréquemment d'environ 10⁻⁴ à environ 1.

De façon plus générale, le catalyseur utilisé dans toute zone d'hydroisomérisation selon le procédé de la présente invention comprend généralement au moins un métal choisi dans le groupe formé par les métaux nobles du groupe VIII de la classification périodique des éléments et le nickel, c'est-à-dire choisi dans le groupe formé par le ruthénium, le rhodium, le palladium, l'osmium, l'iridium, le platine, de préférence le palladium, ou le nickel, utilisé tel quel ou de préférence déposé sur un support. Le métal doit généralement se trouver sous forme réduite au moins pour 50% en poids de sa totalité. Dans le cas de l'utilisation de nickel, la proportion de nickel par rapport au poids total de catalyseur est comprise entre 5 et 70%, et de façon préférée entre 10 et 70 %. De plus on utilise généralement un catalyseur tel que la taille moyenne des cristallites de nickel est inférieure à 10 nm, de préférence inférieure à 8 nm, de façon encore plus préférée inférieure à 6 nm. Mais tout autre catalyseur d'hydroisomérisation connu de l'homme du métier peut également être choisi. Le catalyseur est habituellement traité par un composé du soufre puis par de l'hydrogène avant son utilisation. Le catalyseur est généralement sulfuré in situ ou ex situ de telle façon que du soufre soit chimisorbé sur une partie au moins du métal. Le soufre chimisorbé a pour effet de favoriser la réaction d'hydroisomérisation du butène-1 en butènes-2 par rapport à la réaction d'hydrogénation de l'isobutène et donc de maximiser le rendement en isobutène du procédé.

Le support du catalyseur d'hydroisomérisation est généralement choisi dans le groupe formé par l'alumine, les silice-alumines, la silice, les zéolithes, le charbon actif, les argiles, les ciments alumineux, les oxydes de terres rares et les oxydes alcalino-terreux, seuls ou en mélange. On utilise de préférence un support à base d'alumine ou de silice, de surface spécifique comprise entre 10 et 300 m²/ g, de préférence entre 30 et 70 m²/g.

A titre d'exemples non limitatifs de catalyseurs utilisables dans le cadre de la présente invention, on peut citer les catalyseurs commerciaux tels que celui vendu par la société Catalysts and Chemicals sous la référence C-31, celui vendu par la société Girdler Corporation sous la référence G-55 ou, de préférence, ceux vendus par la société Procatalyse sous les références LD-265, LD-265S, LD-267 et LD-267R.

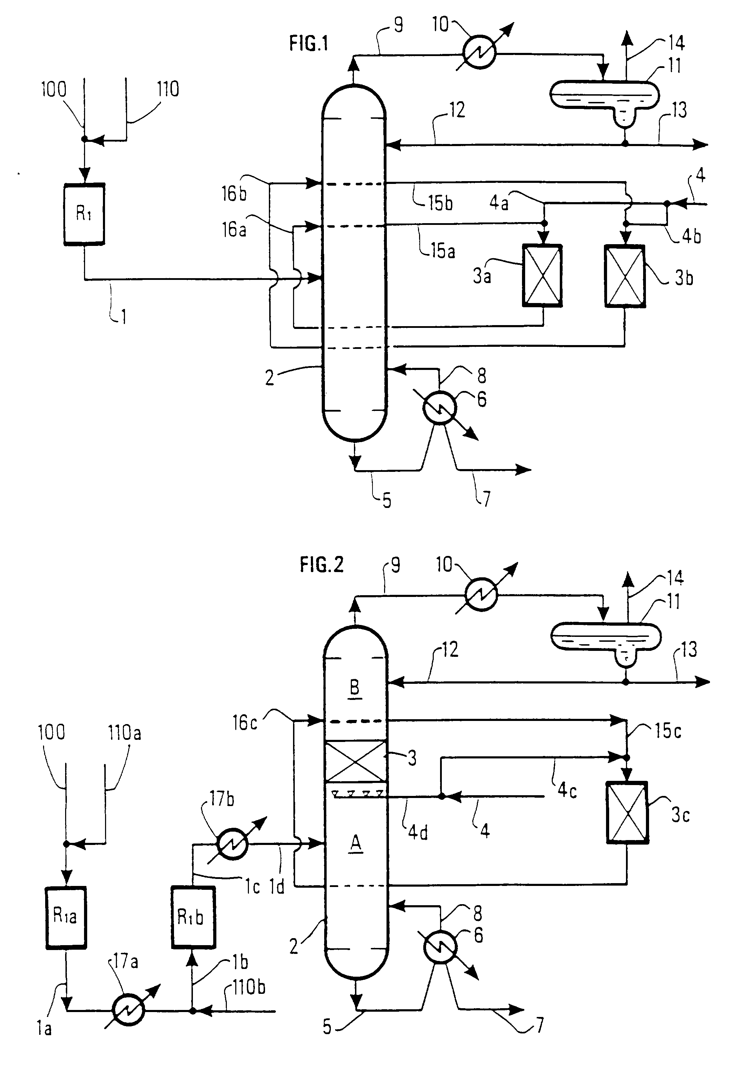

Les figures 1 et 2 constituent chacune une illustration d'une possibilité de réalisation du procédé selon l'invention. Les dispositifs similaires sont représentés par les mêmes chiffres sur ces figures. Les appareillages tels que les pompes ou les vannes n'ont pas été représentés.

Une première réalisation du procédé est représentée sur la figure 1. La coupe C₄ brute, après extraction de la majeure partie du butadiène (coupe appelée raffinat-1), contenant au moins de l'isobutène et du butène-1, est envoyée dans un réacteur R1 d'hydroisomérisation-hydrogénation par la ligne 100 en mélange avec de l'hydrogène introduit par la ligne 110. La quantité d'hydrogène est ajustée en particulier en fonction de la teneur de la charge en composés multi-insaturés tels que le butadiène, le vinylacétylène, le méthylacétylène et l'éthylacétylène, de manière avoir un excès suffisant pour effectuer hydrogénation et l'hydroisomérisation au moins partielle du butène-1 en butènes-2. Le produit obtenu à la sortie du réacteur R1 est envoyé par la ligne 1 dans la colonne 2. Ladite colonne contient des internes de distillation, qui sont, par exemple dans le cas représenté sur la figure 1, des plateaux ou du garnissage, représentés en partie par des traits pointillés sur ladite figure.

Dans certains cas, quand la teneur de la coupe C₄ en composés insaturés est trop élevée, une partie de l'effluent du réacteur R1, après échange thermique, peut être recyclée dans le réacteur R1 avant de recevoir de l'hydrogène par la ligne 110. Le recyclage d'une partie de l'effluent du réacteur R1 appauvri en composés polyinsaturés permet d'abaisser la teneur en composés polyinsaturés dans la charge du réacteur R1. La ligne de recyclage et les éventuels équipements associés ne sont pas représentés sur la figure 1.

En pied de colonne, la fraction la moins volatile, constituée principalement par les butènes-2, est récupérée par la ligne 5, rebouillie dans l'échangeur 6 et évacuée par la ligne 7. La vapeur de rebouillage est réintroduite dans la colonne par la ligne 8. En tête de colonne, la fraction la plus volatile, c'est-à-dire comprenant principalement de l'isobutène et de l'isobutane, est envoyée par la ligne 9 dans un condenseur 10 puis dans un ballon 11 où intervient une séparation entre une phase liquide et une phase vapeur comprenant principalement des hydrocarbures légers C₃⁻ et de l'hydrogène éventuellement en excès. La phase vapeur est évacuée du ballon par la ligne 14. La phase liquide du ballon 11 est renvoyée pour partie, par la ligne 12, en tête de colonne pour en assurer le reflux, tandis que l'autre partie constitue le distillat liquide très appauvri en n-butènes qui est évacué par la ligne 13.

Au moyen d'un plateau de soutirage disposé dans la zone de rectification de la colonne, on soutire par la ligne 15a un liquide que l'on envoie en tête d'un réacteur d'hydroisomérisation 3a, après adjonction d'hydrogène par les lignes 4 puis 4a. L'effluent du réacteur d'hydroisomérisation est recyclé à la colonne par la ligne 16a, sensiblement au niveau de la ligne 15a de prélèvement.

De même, on soutire par la ligne 15b un liquide que l'on envoie dans le réacteur d'hydroisomérisation 3b, après adjonction d'hydrogène par les lignes 4 et 4b, et l'on recycle à la colonne par la ligne 16b (très légèrement au dessus du niveau de prélèvement 15b) l'effluent du réacteur d'hydroisomérisation 3b.

Selon un deuxième mode de réalisation du procédé, représenté sur la figure 2, la coupe C4 brute de vapocraquage est envoyée dans un réacteur R1a d'hydroisomérisation-hydrogénation, par la ligne 100, en mélange avec de l'hydrogène introduit par la ligne 110a. L'effluent du réacteur R1a est envoyé dans l'échangeur de refroidissement 17a par la ligne la, puis dans un second réacteur R1b d'hydroisomérisation par la ligne lb, en mélange avec de l'hydrogène introduit par la ligne 110b. L'effluent du réacteur R1b est envoyé dans l'échangeur de refroidissement 17b par la ligne 1c, puis, via la ligne 1d, dans une colonne de distillation 2, munie d'internes de distillation, qui sont, par exemple dans le cas de la figure 2, des plateaux de distillation multi-déversoirs, ainsi que d'un interne catalytique 3 contenant un catalyseur d'hydroisomérisation et alimenté par de l'hydrogène par les lignes 4 puis 4d.

Dans certains cas, quand la teneur de la coupe C4 en composés insaturés est trop élevée, une partie de l'effluent du réacteur R1a, après échange thermique par exemple dans l'échangeur 17a, peut être recyclée dans le réacteur R1a avant de recevoir de l'hydrogène par la ligne 110a. Le recyclage d'une partie de l'effluent du réacteur R1a appauvri en composés polyinsaturés permet d'abaisser la teneur en composés polyinsaturés dans la charge du réacteur R1a. La ligne de recyclage et les éventuels équipeemnts associés ne sont pas représentés sur la figure 2.

Les effluents de tête et de fond de la colonne sont traités comme décrit ci-dessus pour la première réalisation du procédé. D'un plateau de soutirage disposé dans la zone de rectification de la colonne, on prélève par la ligne 15c un liquide qui, après adjonction d'hydrogène par la ligne 4c, est introduit dans le réacteur d'hydroisomérisation 3c. L'effluent du réacteur d'hydroisomérisation est recyclé à la colonne de distillation par la ligne 16c, à un niveau représenté sensiblement au niveau de soutirage 15c du liquide.

Selon un troisième mode de réalisation du procédé, très proche du deuxième mode de réalisation qui est représenté sur la figure 2, le plateau de soutirage en liaison avec les lignes 15c et 16 est situé au-dessous de l'élément interne catalytique 3, toutes choses étant égales par ailleurs.

Les exemples qui suivent illustrent l'invention de manière non limitative. Il doit être entendu que d'autres arrangements des réacteurs, des échangeurs etc. que ceux décrits peuvent être envisagés à titre de variantes de l'invention, du moment qu'ils entrent dans la définition du procédé.

### Exemple 1 :

Les opérations d'hydroisomérisation d'une coupe C4 et de distillation ont été effectuée successivement de façon discontinue. La charge a été hydroisomérisée une première fois. L'effluent de ce premier essai a été distillée ; la tête de distillation, représentative d'un soutirage intermédiaire a été hydroisomérisée. L'effluent de l'hydroisomérisation, représentative de ce qui serait réinjecté dans la colonne, a été distillée. La tête de cette seconde distillation a été hydroisomérisée, et l'effluent de cette troisième hydroisomérisation a été distillé.

Les opérations d'hydroisomérisation sont effectuées dans une unité pilote disposant d'un réacteur adiabatique. Le réacteur est remplie de 1,5 1 du catalyseur LD-265 vendu par la société Procatalyse. Le catalyseur est sulfuré et activé in situ selon une procédure préconisée par le fournisseur du catalyseur.

Les opérations de distillation ont été effectuées dans une colonne adiabatique de diamètre interne 163 mm et de hauteur de 10 m. La colonne est constituée de 4 lits de 1,78 m de haut au dessus de l'injection de la charge, remplis d'un garnissage commercialisé par la société Sulzer sous le nom de M550Y et de 2 lits de 1 m de hauteur en dessous de l'injection de la charge, remplis d'anneaux de Pall.

### - Première hydroisomérisation.

Les conditions opératoires moyennes au cours de l'essai sont les suivantes :
- Température du réacteur : 80°C
- Pression du réacteur : 20 bar
- Temps de séjour : 0,25 h.
- Rapport molaire H₂/charge : 3

Le tableau 1 ci-après montre les compositions de la charge et de l'effluent du réacteur d'hydroisomérisation fonctionnant dans les conditions décrites ci-dessus.

**TABLEAU 1**

| | Charge (% poids) | Effluent (% poids) |
|---|---|---|
| < C₄ | 0,25 | 0,23 |
| i C₄ | 2,98 | 3,10 |
| i C₄⁼ | 44,90 | 44,42 |
| C₄⁼ 1 | 26,95 | 4,26 |
| C₄⁼⁼ 1,3 | 0,13 | 0,00 |
| n C₄ | 11,72 | 14,41 |
| C₄⁼ 2 trans | 8,73 | 21,37 |
| Néo C₅ | 0,24 | 0,23 |
| Me Cyclo C₃ | 0,06 | 0,06 |
| C₄⁼ 2 cis | 4,03 | 11,92 |
| > C₄ | 0,01 | 0,00 |

avec la légende suivante pour ce tableau et pour les tableaux suivants :
- < C₄ :: composés à moins de 4 (4 exclu) atomes de carbone par molécule (ou C3⁻)
- iC₄ :: isobutane
- i C₄⁼ :: isobutène
- C₄⁼ :: 1 butène-1
- C₄⁼⁼ :: 1,3 butadiène-1,3
- n C₄ :: normal-butane
- C₄⁼ :: 2 trans butène-2 trans
- Néo C₅ :: néopentane (ou diméthyl propane)
- Me Cyclo C₃ :: méthyl cyclopropane
- C₄⁼ 2 cis :: butène-2 cis
- > C₄ :: composés à plus de 4 (4 exclu) atomes de carbone par molécule (ou C5⁺)

### - Première distillation.

La distillation de l'effluent de l'essai présenté ci dessus s'est effectué avec les conditions opératoires suivantes :
- Pression de la colonne : 4 bar
- Taux de reflux (R/D) : 20
- Température de la charge : 33°C
- Température de reflux : 32°C
- Température en tête de colonne : 57°C
- Température en fond de colonne : 63°C.

Le tableau 2 ci-après montre les compositions de la charge et de l'effluent de tête de la colonne de distillation fonctionnant dans les conditions décrites ci-dessus.

**TABLEAU 2**

| | Charge (% poids) | Tête (% poids) |
|---|---|---|
| < C₄ | 0,23 | 0,44 |
| i C₄ | 3,10 | 6,71 |
| i C₄⁼ | 44,42 | 83,35 |
| C₄⁼ 1 | 4,26 | 7,39 |
| C₄⁼⁼ 1,3 | 0,00 | 0,00 |
| n C₄ | 14,41 | 1,62 |
| C₄⁼ 2 trans | 21,37 | 0,44 |
| Néo C₅ | 0,23 | - |
| Me Cyclo C₃ | 0,06 | - |
| C₄⁼ 2 cis | 11,92 | 0,05 |
| > C₄ | - | - |

### - Deuxième hydroisomérisation.

Les conditions opératoires moyennes au cours de l'essai sont les suivantes :
- Température du réacteur : 65°C
- Pression du réacteur : 20 bar
- Temps de séjour : 0,25 h
- Rapport molaire H₂/charge : 0,6

Le tableau 3 ci-après montre les compositions de la charge et de l'effluent du réacteur d'hydroisomérisation fonctionnant dans les conditions décrites ci-dessus.

**TABLEAU 3**

| | Charge (% poids) | Effluent (% poids) |
|---|---|---|
| < C₄ | 0,44 | 0,39 |
| i C₄ | 6,71 | 6,91 |
| i C₄⁼ | 83,35 | 82,94 |
| C₄⁼ 1 | 7,39 | 0,81 |
| C₄⁼⁼ 1,3 | - | - |
| n C₄ | 1,62 | 2,09 |
| C₄⁼ 2 trans | 0,44 | 4,44 |
| Néo C₅ | - | - |
| Me Cyclo C₃ | - | - |
| C₄⁼ 2 cis | 0,05 | 2,42 |
| > C₄ | - | - |

### - Deuxième distillation.

La distillation de l'effluent de l'essai présenté ci dessus s'est effectué avec les conditions opératoires suivantes :
- Pression de la colonne : 4 bar
- Taux de reflux (R/D) : 13,5
- Température de la charge : 36°C
- Température de reflux : 41°C
- Température en tête de colonne : 51°C
- Température en fond de colonne : 55°C.

Le tableau 4 ci-après montre les compositions de la charge et de l'effluent de tête de la colonne de distillation fonctionnant dans les conditions décrites ci-dessus.

**TABLEAU 4**

| | Charge (% poids) | Tête (% poids) |
|---|---|---|
| < C₄ | 0,39 | 0,65 |
| i C₄ | 6,91 | 13,71 |
| i C₄⁼ | 82,94 | 84,82 |
| C₄⁼ 1 | 0,81 | 0,51 |
| C₄⁼⁼ 1,3 | - | - |
| n C₄ | 2,09 | 0,14 |
| C₄⁼ 2 trans | 4,44 | 0,12 |
| Néo C₅ | - | - |
| Me Cyclo C₃ | - | - |
| C₄⁼ 2 cis | 2,42 | 0,05 |
| > C₄ | - | - |

### - Troisième hydroisomérisation.

Les conditions opératoires moyennes au cours de l'essai sont les suivantes :
- Température du réacteur : 60°C
- Pression du réacteur : 20 bar
- Temps de séjour : 0,25 à 0,1 h
- Rapport molaire H₂/charge : 1

Le tableau 5 ci-après montre les compositions de la charge et de l'effluent du réacteur d'hydroisomérisation fonctionnant dans les conditions décrites ci-dessus.

**TABLEAU 5**

| | Charge (% poids) | Effluent (% poids) |
|---|---|---|
| < C₄ | 0,65 | 0,57 |
| i C₄ | 13,71 | 14,55 |
| i C₄⁼ | 84,82 | 84,07 |
| C₄⁼ 1 | 0,51 | 0,03 |
| C₄⁼⁼ 1,3 | - | - |
| n C₄ | 0,14 | 0,22 |
| C₄⁼ 2 trans | 0,12 | 0,38 |
| Néo C₅ | - | - |
| Me Cyclo C₃ | - | 1 - |
| C₄⁼ 2 cis | 0,05 | 0,18 |
| > C₄ | - | - |

### - Troisième distillation.

La distillation de l'effluent de l'essai présenté ci dessus s'est effectué avec les conditions opératoires suivantes :
- Pression de la colonne : 4 bar
- Taux de reflux (R/D) : 13,5
- Température de la charge : 36°C
- Température de reflux : 41°C
- Température en tête de colonne : 53°C
- Température en fond de colonne : 55°C.

Le tableau 6 ci-après montre les compositions de la charge et de l'effluent de tête de la colonne de distillation fonctionnant dans les conditions décrites ci-dessus.

**TABLEAU 6**

| | Charge (% poids) | tête (% poids) |
|---|---|---|
| < C ₄ | 0,57 | 0,57 |
| i C₄ | 14,55 | 14,66 |
| i C₄⁼ | 84,07 | 84,69 |
| C₄⁼ 1 | 0,03 | 0,03 |
| C₄⁼⁼ 1,3 | - | - |
| n C₄ | 0,22 | 0,01 |
| C₄⁼ 2 trans | 0,38 | 0,04 |
| Néo C₅ | - | - |
| Me Cyclo C₃ | - | - |
| C₄⁼ 2 cis | 0,18 | - |
| > C₄ | - | - |

Ces opérations successives et discontinues d'hydroisomérisation et de distillation représentent l'opération de séparation du butène-1 de l'isobutène qui est effectuée en continu dans le cas du procédé selon l'invention.

### Exemple 2 :

Des tests pilotes d'hydroisomérisation ont été effectués à partir d'un raffinat-1 sur le catalyseur d'hydroisomérisation LD267R commercialisé par la société Procatalyse. Les résultats de ces tests sont présentés dans le tableau 7 ci dessous ; ils ont permis de déterminer les paramètres de calcul qui permettent de simuler le procédé selon l'invention au moyen de logiciels adaptés. Le logiciel utilisé pour cette simulation est commercialisé sous le nom de Pro2 par la société SIMCI.

**TABLEAU 7 :**

| résultats de tests pilotes | | | | | | | | | | | |
|---|---|---|---|---|---|---|---|---|---|---|---|
| T °C | | 40 | 60 | 90 | 50 | 50 | 50 | 50 | 50 | 50 | 50 |
| VVH h-1 | | 30 | 30 | 30 | 30 | 30 | 30 | 30 | 30 | 20 | 40 |
| P bar | | 10 | 10 | 10 | 6,5 | 10 | 15 | 10 | 10 | 10 | 10 |
| H2/ HC m/m | | 0,17 | 0,17 | 0,17 | 0,17 | 0,17 | 0,17 | 0,1 | 0,19 | 0,17 | 0,17 |
| | char | effl | effl | effl | effl | effl | effl | effl | effl | effl | effl |
| < C₄ | 0,14 | 0,11 | 0,12 | 0,11 | 0,10 | 0,10 | 0,10 | 0,10 | 0,11 | 0,10 | 0,09 |
| iC4 | 5,69 | 5,75 | 5,75 | 5,73 | 5,71 | 5,76 | 5,75 | 5,72 | 5,76 | 5,75 | 5,74 |
| iC4= | 78,67 | 78,71 | 78,72 | 78,73 | 78,78 | 78,72 | 78,73 | 78,74 | 78,72 | 78,71 | 78,74 |
| 1-C4= | 3,66 | 1,30 | 0,91 | 0,75 | 1,15 | 1,01 | 1,18 | 1,13 | 1,00 | 0,8 | 1,32 |
| n-C4 | 7,16 | 7,19 | 7,17 | 7,14 | 7,14 | 7,18 | 7,19 | 7,16 | 7,20 | 7,19 | 7,18 |
| tr2-C4= | 4,36 | 5,40 | 5,48 | 5,40 | 5,46 | 5,49 | 5,41 | 5,46 | 5,48 | 5,59 | 5,37 |
| cs2-C4= | 0,32 | 1,54 | 1,85 | 2,14 | 1,66 | 1,74 | 1,64 | 1,69 | 1,75 | 1,86 | 1,56 |
| où char = charge et effl = effluent | | | | | | | | | | | |

Deux exemples ainsi simulés par le calcul ont été effectués. Ils sont décrits ci-après.

### Exemple 2A :

La configuration de l'unité, comportant trois zones d'hydroisomérisation situées à l'extérieur de la colonne, est la suivante :
- colonne de 130 plateaux théoriques, numérotés de haut en bas,
- plateau d'alimentation n° 90,
- les réacteurs extérieurs sont alimentés par des soutirages situés respectivement aux plateaux 10, 25 et 39. L'effluent de chacun des réacteurs extérieurs est réintroduit sur le plateau de soutirage alimentant le réacteur extérieur considéré.

### Réacteurs :

Les trois réacteurs contiennent chacun 7,5 tonnes de catalyseur.

### Conditions opératoires :

taux de reflux : 12,
pression en tête de colonne : 6,2 bar absolu,
pression en fond de colonne : 7 bar absolu,
température de l'alimentation de la colonne : 59 °C,
température en tête de colonne : 45 °C,
température en fond de colonne : 64,5 °C,
température du réacteur alimenté par un soutirage au plateau 10 : 53 °C,
pression du réacteur alimenté par un soutirage au plateau 10 : 6,6 bar absolu,
débit dans le réacteur alimenté par un soutirage au plateau 10: 2800 kmole/h,
température du réacteur alimenté par un soutirage au plateau 25 : 54 °C,
pression du réacteur alimenté par un soutirage au plateau 25 : 6,6 bar absolu,
débit dans le réacteur alimenté par un soutirage au plateau 25: 2800 kmole/h,
température du réacteur alimenté par un soutirage au plateau 39 : 55 °C,
pression du réacteur alimenté par un soutirage au plateau 39 : 6,7 bar absolu,
débit dans le réacteur alimenté par un soutirage au plateau 39: 2800 kmole/h.

Avec cette configuration et sous ces conditions opératoires, la simulation a conduit aux résultats suivants :

| | alimentation colonne (kmole/h) | tête de colonne (kmole/h) | fond de colonne (kmole/h) |
|---|---|---|---|
| < C₄ | 1,12 | 1,12 | 0,00 |
| i C₄ | 4,46 | 5,32 | 0,00 |
| i C₄⁼ | 110,08 | 108,33 | 0,89 |
| C₄⁼ 1 | 7,53 | 0,03 | 0,19 |
| n C₄ | 55,27 | 0,67 | 54,66 |
| C₄⁼ 2 tr | 79,73 | 0,07 | 84,50 |
| C₄⁼ 2 cis | 33,49 | 0,00 | 35,90 |
| H₂ | 1,20 | 0,27 | 0,00 |
| Total | 292,88 | 115,81 | 176,14 |

Rendement en isobutène en tête de colonne : 98,4 %
Rapport molaire butène-1/isobutène en tête de colonne : 2,66 x 10⁻⁴.

### Exemple 2B:

La configuration de l'unité, comportant deux zones d'hydroisomérisation à l'intérieur de la colonne et une zone d'hydroisomérisation à l'extérieur, est la suivante :
- 130 plateaux théoriques, numérotés de haut en bas,
- plateau d'alimentation n° 90,
- plateaux réactifs 10 et 25,
- le réacteur extérieur est alimenté par un soutirage situé au plateau 39. L'effluent du réacteur extérieur est réintroduit sur le plateau 39 de soutirage alimentant le réacteur extérieur.

### Réacteurs :

le réacteur en amont de la colonne contient 7,5 m³ de catalyseur, les plateaux réactifs 10 et 25 et le réacteur extérieur contiennent chacun 7,5 m³ de catalyseur.

### Conditions opératoires :

taux de reflux : 12,
pression en tête de colonne : 6,2 bar absolu,
pression en fond de colonne : 7 bar absolu,
température de l'alimentation de la colonne : 59 °C,
température en tête de colonne : 47,5 °C,
température en fond de colonne : 64,6 °C,
température du plateau réactif10 : 53 °C,
pression du plateau réactif 10 : 6,6 bar absolu,
débit dans le plateau réactif 10 : 1555 kmole/h
température du plateau réactif 25 : 54 °C,
pression du plateau réactif 25 : 6,6 bar absolu,
débit dans le plateau réactif 25 : 1558 kmole/h,
température du réacteur alimenté par un soutirage au plateau 39 : 55 °C,
pression du réacteur alimenté par un soutirage au plateau 39 : 6,7 bar absolu,
débit dans le réacteur alimenté par un soutirage au plateau 39 : 2800 kmole/h.

Avec cette configuration et sous ces conditions opératoire, la simulation a conduit aux résultats suivants :

| | alimentation colonne (kmole/h) | tête de colonne (kmole/h) | fond de colonne (kmole/h) |
|---|---|---|---|
| < C₄ | 1,12 | 1,12 | 0,00 |
| i C₄ | 4,46 | 5,31 | 0,00 |
| i C₄⁼ | 110,08 | 108,34 | 0,89 |
| C₄⁼ 1 | 7,53 | 0,05 | 0,19 |
| n C₄ | 55,27 | 1,03 | 54,45 |
| C₄⁼ 2 tr | 79,75 | 0,10 | 84,40 |
| C₄⁼ 2 cis | 33,49 | 0,01 | 35,89 |
| H₂ | 1,20 | 0,14 | 0,00 |
| Total | 292,88 | 116,1 | 175,82 |

Rendement en isobutène en tête de colonne : 98,4 %
Rapport molaire butène-1/isobutène en tête de colonne : 4,23 x 10⁻⁴.

## Revendications

1. Procédé de traitement d'une charge, comprenant en majeure partie des hydrocarbures oléfiniques comportant 4 atomes de carbone par molécule, dont de l'isobutène, ainsi que du butène-1 et des butènes-2 en rapport correspondant sensiblement à l'équilibre thermodynamique, dans lequel on traite ladite charge dans une zone de distillation, associée à une zone réactionnelle d'hydroisomérisation, au moins en partie externe à la zone de distillation, ledit procédé étant caractérisé en ce que la charge de la zone réactionnelle est prélevée à la hauteur d'un niveau de prélèvement de la zone de distillation et représente au moins une partie du liquide coulant dans la zone de distillation ; l'effluent de ladite zone réactionnelle étant au moins en partie réintroduit dans la zone de distillation à au moins un niveau de réintroduction, de manière à assurer la continuité de la distillation.

2. Procédé selon la revendication 1 tel que le niveau de réintroduction est situé à proximité dudit niveau de prélèvement.

3. Procédé selon l'une des revendications 1 ou 2 tel que le niveau de réintroduction est situé à une distance dudit niveau de prélèvement correspondant à une hauteur comprise entre 0 et 4 plateaux théoriques au-dessus ou au-dessous dudit niveau de prélèvement.

4. Procédé selon l'une des revendications 1 à 3 tel que la charge est obtenue à partir d'une coupe comprenant en majeure partie des hydrocarbures oléfiniques comportant 4 atomes de carbone par molécule par traitement de ladite coupe dans une première zone d'hydroisomérisation.

5. Procédé selon l'une des revendications 1 à 4 tel que l'on procède à l'alimentation de la zone de distillation, en plus de l'alimentation en l'effluent qui constitue la charge principale, en une charge secondaire qui est une coupe oléfinique C₄ contenant au moins de l'isobutène, ainsi que du butène-1 et des butènes-2 en rapport correspondant sensiblement à l'équilibre thermodynamique.

6. Procédé selon l'une des revendications 1 à 5 comportant de 1 à 6 niveau(x) de prélèvement.

7. Procédé selon l'une des revendications 1 à 6 tel que la distillation est réalisée sous une pression comprise entre 2 et 30 bar, avec un taux de reflux compris entre 1 et 30, la température de tête de zone de distillation étant comprise entre 0 et 200°C et la température de fond de zone de distillation étant comprise entre 5 et 250°C.

8. Procédé selon l'une des revendications 1 à 7 dans lequel la zone réactionnelle d'hydroisomérisation associée à la zone de distillation est en totalité externe à la zone de distillation.

9. Procédé selon l'une des revendications 1 à 7 dans lequel la zone d'hydroisomérisation associée à la zone de distillation est à la fois partiellement incorporée dans la zone de distillation et partiellement externe à la zone de distillation.

10. Procédé selon l'une des revendications 1 à 9 dans lequel toute zone d'hydroisomérisation est telle que toute réaction d'hydroisomérisation est réalisée en présence d'un catalyseur d'hydroisomérisation et d'un flux gazeux comprenant de l'hydrogène.

11. Procédé selon l'une des revendications 1 à 10 tel que, pour la première zone d'hydroisomérisation éventuelle et pour la partie de la zone d'hydroisomérisation associée à la zone de distillation externe à la zone de distillation, la pression requise pour cette étape d'hydroisomérisation est comprise entre 1 et 40 bar, la température est comprise entre 20 et 150°C, et la vitesse spatiale au sein de ladite partie, calculée par rapport au catalyseur, est généralement d'environ 1 à 100 h⁻¹ (volume de charge par volume de catalyseur et par heure).

12. Procédé selon l'une des revendications 1 à 11 tel que tout lit catalytique de la partie externe de la zone d'hydroisomérisation associée à la zone de distillation est alimenté par au moins une partie du liquide coulant sur un plateau de la zone de distillation au niveau de prélèvement qui sert à alimenter ledit lit, et on peut réinjecter l'effluent dudit lit sur un plateau situé à une distance dudit plateau théorique de prélèvement entre 0 et 4 plateaux théoriques.

13. Procédé selon l'une des revendications 1 à 12 tel que le débit de charge d'un réacteur de la partie externe de la zone d'hydroisomérisation associée à la zone de distillation au niveau de prélèvement alimentant ledit réacteur, est compris entre 0,5 et 1,5 fois le débit de liquide coulant sur le plateau associé audit niveau de prélèvement.

14. Procédé selon l'une des revendications 1 à 13 tel que le catalyseur utilisé dans toute zone d'hydroisomérisation, contenant au moins un métal noble du groupe VIII choisi dans le groupe formé par le ruthénium, le rhodium, le palladium, l'osmium, l'iridium et le platine, ou le nickel, est traité par un composé contenant du soufre, puis par de l'hydrogène avant son utilisation.

## Patentansprüche

1. Verfahren zum Behandeln einer Charge, die zum größeren Teil olefinische Kohlenwasserstoffe mit 4 Kohlenstoffatomen pro Molekül, darunter das Isobuten sowie Buten-1 und Butene-2 umfaßt, in einem Anteil, der im wesentlichen dem thermodynamischen Gleichgewicht entspricht, bei dem man diese Charge in eine Destillationszone, zugeordnet zu einer Isomerisierungsreaktionszone behandelt, die wenigstens zum Teil außerhalb der Destillationszone sich befindet, dadurch gekennzeichnet, dass die Charge der Reaktionszone in Höhe eines Entnahmeniveaus der Destillationszone entnommen wird und wenigstens einen Teil der in der Destillationszone strömenden Flüssigkeit darstellt; wobei der Abstrom aus dieser Destillationszone wenigstens zum Teil wieder in die Destillationszone auf wenigstens einem Wiedereinführungsniveau eingeführt wird, derart, dass die Destillationskontinuität sichergestellt ist.

2. Verfahren nach Anspruch 1, derart, dass das Wiedereinführungsniveau benachbart diesem Entnahmeniveau sich befindet.

3. Verfahren nach einem der Ansprüche 1 oder 2, derart, dass das Wiedereinführungsniveau sich unter einer Entfernung von diesem Entnahmeniveau befindet, das einer Höhe zwischen 0 und 4 theoretischen Böden oberhalb oder unterhalb dieses Entnahmeniveaus entspricht.

4. Verfahren nach einem der Ansprüche 1 bis 3, derart, dass die Charge erhalten wird aus einem Schnitt, der zum größeren Teil olefinische Kohlenwasserstoffe mit 4 Kohlenstoffatomen pro Molekül durch Behandlung dieses Schnitts in einer ersten Hydroisomerierungszone umfasst.

5. Verfahren nach einem der Ansprüche 1 bis 4, derart, dass man die Speisung der Destillationzone zusätzlich zur Speisung mit Abstrom, der die Hauptcharge darstellt, mit einer Sekundärcharge vornimmt, die ein olefinischer C₄-Schnitt ist, der wenigstens Isobuten sowie Butene-1 und Buten-2 in einem Anteil entsprechend im wesentlichen dem thermodynamischen Gleichgewicht enthält.

6. Verfahren nach einem der Ansprüche 1 bis 5, 1 bis 6 Entnahmeniveaus enthaltend.

7. Verfahren nach einem der Ansprüche 1 bis 6, derart, dass die Destillation unter einem Druck zwischen 2 und 30 bar mit einem Rückflussgrad zwischen 1 und 30 realisiert wird, wobei die Kopftemperatur der Destillationszone zwischen 0 und 200°C und die Bodentemperatur der Destillationszone zwischen 5 und 250°C liegt.

8. Verfahren nach einem der Ansprüche 1 bis 7, bei dem die Isomerierungsreaktionszone, zugeordnet zur Destillationszone, insgesamt außerhalb der Destillationszone sich befindet.

9. Verfahren nach einem der Ansprüche 1 bis 7, bei dem die Hydroisomerisierungszone, zugeordnet zur Destillationszone gleichzeitig teilweise in die Destillationszone und teilweise außerhalb der Destillationszone eingebaut ist.

10. Verfahren nach einem der Ansprüche 1 bis 9, bei dem jede Hydroisomerisierungszone derart ist, dass jede Hydroisomerisierungsreaktion in Anwesenheit eines Hydroisomerisierungskatalysators und eines Wasserstoff enthaltenden Gasstroms durchgeführt wird.

11. Verfahren nach einem der Ansprüche 1 bis 10, derart, dass für die erste eventuelle Hydroisomerisierungszone und für den Teil der Hydroisomerisierungszone, der der Destillationszone außerhalb der Destillationszone zugeordnet ist, der für diese Hydroisomerisierungsstufe erforderliche Druck zwischen 1 und 40 bar, die Temperatur zwischen 20 und 150°C und, die räumliche Geschwindigkeit inmitten dieses Teils, berechnet in Bezug auf den Katalysator im allgemeinen, bei etwa 1 bis 100 h⁻¹ (Volumen Charge pro Volumen Katalysator und Stunde) liegt.

12. Verfahren nach einem der Ansprüche 1 bis 11, derart, dass jedes katalytische Bett des Teils außerhalb der Hydroisomerisierungszone, zugeordnet zur Destillationszone, gespeist wird durch wenigstens einen Teil, der auf einem Boden der Destillationszone in Entnahmehöhe strömenden Flüssigkeit, die dazu dient dieses zu speisen und dass man den Abstrom aus diesem Bett auf einen Boden wieder einspritzen kann, der unter einer Entfernung vom theoretischen Entnahmeboden zwischen 0 und 4 theoretischen Böden sich befindet.

13. Verfahren nach einem der Ansprüche 1 bis 12, derart, dass der Chargendurchsatz eines Reaktors des Teils außerhalb der Hydroisomerisierungszone, zugeordnet zur Destillationszone in Höhe der diesen Reaktor speisenden Entnahme zwischen dem 0,5 und 1,5-fachen des Flüssigkeitsdurchsatzes, der auf dem diesem Entnahmeniveau zugeordneten Boden strömt, liegt.

14. Verfahren nach einem der Ansprüche 1 bis 13, derart, dass der in jeder Hydroisomerisierungszone verwendete Katalysator der wenigstens ein Edelmetall der Gruppe VIII enthält, die gewählt ist aus der durch Ruthenium, Rhodium, Palladium, Osmium, Iridium und Platin oder Nickel gebildeten Gruppe, durch eine Schwefel enthaltende Verbindung, dann durch Wasserstoff vor seiner Verwendung behandelt wird.

## Claims

1. A process for treating a feedstock comprising a major part of the olefinic hydrocarbons having 4 carbon atoms per molecule, including isobutene as well as but-1-ene and but-2-enes in a ratio corresponding substantially to thermodynamic equilibrium, wherein the process comprises processing said feedstock in a distillation zone associated with a hydroisomerization reaction zone, at least partly external to the distillation zone, said process being characterized in that the feedstock of the reaction zone is drawn off at the height of a draw-off level of the distillation zone, and represents at least part of the liquid flowing in the distillation zone ; at least part of the effluent of said reaction zone being reintroduced into the distillation zone at one or more reintroduction level(s), so as to ensure the continuity of the distillation.

2. A process as claimed in claim 1 wherein the reintroduction level is located in proximity to said draw-off level.

3. A process as claimed in any one of claims 1 or 2 wherein the reintroduction level is located at a distance from said draw-off level corresponding to a height in the range of 0 to 4 theoretical plates above or below said draw-off level.

4. A process as claimed in any one of claims 1 to 3 wherein the feedstock is produced from a cut comprising for the major part thereof olefinic hydrocarbons comprising 4 carbon atoms per molecule by treating said cut in a first hydroisomerisation zone.

5. A process as claimed in any one of claims I to 4 wherein the distillation zone is supplied, in addition to the supply of the effluent which constitutes the main feedstock, with a secondary feedstock which is a C₄ olefinic cut containing at least isobutene as well as but-1-ene and but-2-enes in a ratio corresponding substantially to thermodynamic equilibrium.

6. A process as claimed in any one of claims 1 to 5 comprising 1 to 6 draw-off level(s).

7. A process as claimed in any one of claims I to 6 wherein the distillation is performed at a pressure in the range of 2 to 30 bars, a reflux ratio in the range of 1 to 30, a temperature at the top of the distillation zone in the range of 0 to 200°C and a temperature at the bottom of the distillation zone in the range of 5 to 250°C.

8. A process as claimed in any one of claims I to 7 wherein the hydroisomerization reaction zone associated with the distillation zone is totally external to the distillation zone.

9. A process as claimed in any one of claims 1 to 7 wherein the hydroisomerization zone associated with the distillation zone is both partly incorporated in the distillation zone and partly external to the distillation zone.

10. A process as claimed in any one of claims I to 9 wherein each hydroisomerization zone is such that any hydroisomerization reaction is carried out in the presence of a hydroisomerization catalyst and a gaseous flow comprising hydrogen.

11. A process as claimed in any one of claims I to 10 wherein for the first optional hydroisomerization zone and for the part of the hydroisomerization zone associated with the distillation zone external to the distillation zone, the pressure of this hydroisomerization stage is in the range of 1 to 40 bars, the temperature is in the range of 20 to 150°C, and the space velocity within said part, calculated in relation to the catalyst, is in the range of from about 1 to 100 h⁻¹ (volume of feed per volume of catalyst and per hour).

12. A process as claimed in any one of claims 1 to 11 wherein any catalyst bed of the external part of the hydroisomerization zone associated with the distillation zone is fed by at least part of the liquid flowing on a plate of the distillation zone at the draw-off level which is used for feeding said bed, and the effluent of said bed is re-injected on a plate located at a distance from said theoretical draw-off plate in the range of 0 to 4 theoretical plates.

13. A process as claimed in any one of claims 1 to 12 in which the flow rate of a reactor of the external part of the hydroisomerization zone associated with the distillation zone, at the draw-off level which feeds said reactor, is in the range of 0.5 to 1.5 time the flow rate of the liquid effluent flowing on said plate, associated to said draw off level.

14. A process as claimed in any one of claims I to 12 in which a supported catalyst is used in any hydroisomerization zones, said catalyst comprising at least one noble metal from group VIII selected from the group consisting of ruthenium, rhodium, palladium, osmium, iridium and platinum, or nickel, processed by a sulfur-containing compound, then by hydrogen prior to being used.
